# EUROPÄISCHE PATENTSCHRIFT

(11) **EP 3 156 519 B1**
(45) Veröffentlichungstag und Bekanntmachung des Hinweises auf die Patenterteilung: **29.08.2018**
(21) Anmeldenummer: 16190444.6
(22) Anmeldetag: 23.09.2016
(51) Int. Cl.: C25B 1/04, C25B 15/08, C07C 1/12, C10G 2/00, C10L 3/08, C04B 2/10

(54) **VERFAHREN UND ANLAGE ZUR ERZEUGUNG EINES KOHLENWASSERSTOFFS**
METHOD AND APPARTUS FOR PRODUCING A HYDROCARBON
PROCÉDÉ ET APPAREIL DE PRODUCTION D'UN HYDROCARBURE

(30) Priorität: 16.10.2015 DE 102015013371; 24.05.2016 DE 102016208938
(43) Veröffentlichungstag der Anmeldung: 19.04.2017
(73) Patentinhaber: Volkswagen Aktiengesellschaft, 38440 Wolfsburg (DE)
(72) Erfinder: Schäfer, Hans-Jürgen, 38112 Braunschweig (DE); Bippes, Michael, 38112 Braunschweig (DE)

(56) Entgegenhaltungen:
- EP-A1- 2 818 227
- WO-A1-2015/033583
- WO-A1-2015/055349
- US-A- 3 817 725
- US-A- 3 938 968
- ALSTOM TECHNOLOGY LTD ET AL: "Process-integrated use of methanation and electrolysis offheat", RESEARCH DISCLOSURE, MASON PUBLICATIONS, HAMPSHIRE, GB, Bd. 594, Nr. 91, 1. Oktober 2013 (2013-10-01), Seite 9, XP007142643, ISSN: 0374-4353

## Beschreibung

Die Erfindung betrifft ein Verfahren sowie eine Anlage zur Erzeugung eines Kohlenwasserstoffs, insbesondere eines Kraftstoffs für Fahrzeuge, aus Kohlenstoffdioxid (nachfolgend Kohlendioxid) CO₂ durch stoffliche und thermische Kopplung zweier Produktionsprozesse.

Die Suche nach alternativen Kraftstoffen für eine auch auf Dekarbonisierung basierende nachhaltige Mobilität hat bisher keine praktikablen Lösungen erbracht, die ausreichende Energiemengen für die Massenmobilität liefern. Dezentrale, lokale/regionale Nischenlösungen und auf Biomasse basierende Ansätze sind langfristig kritisch zu bewerten, da im zunehmenden Anbauflächenwettbewerb Nahrungs- und Futtermittel Priorität haben werden und Kollateralschäden wie die Regenwaldzerstörung inakzeptabel sind.

Auf der anderen Seite wird Kohlendioxid in verschiedenen Industriezweigen in großen Mengen als Nebenprodukt produziert und emittiert. So entstehen bei dem "Entsäuerungsprozess" (Kalzination) von Kalkstein CaCO₃ für die Erzeugung der Wirtschaftsgüter Zement und Kalk gewaltige CO₂-Prozessemissionen (2008 in Deutschland: insgesamt 21 Mio. t CO₂ beziehungsweise pro Standort ca. 1 Mio. t CO₂). Das gleiche gilt für die Eisen/Stahl-Industrie (2009 in Deutschland 52,3 Mio. t CO₂ beziehungsweise pro Hochofen im Mittel 5,5 Mio. t CO₂). Diese beiden Industrien zeichnen sich zudem durch relativ hohe CO₂-Anteile im Abgas aus, nämlich durch einen CO₂-Anteil im Abgas der Zementproduktion zwischen 20 und 30 Vol.-% und 27 Vol.-% bei der Stahlproduktion gegenüber nur 14 % als typische CO₂-Konzentration im Abgas eines Kohlekraftwerkes. Diese hohen Anteile mindern die Kosten für die CO₂-Abtrennung. Wünschenswert ist, diese Emissionen möglichst effizient für weitere Verarbeitungsprozesse aufzufangen und weiter zu verwerten.

In WO 2010/069 622 A1 ist die Nutzung von Kohlendioxid CO₂ als Rohstoff zur Herstellung eines möglichst nachhaltig erzeugten synthetischen Kraftstoffs beschrieben. Hierzu wird Wasserstoff elektrolytisch unter Verbrauch einer Kombination von regenerativem und konventionellem Strom erzeugt und mit CO₂ zu Methanol oder einem anderen Kohlenwasserstoff umgesetzt. Das CO₂ wird aus dem Rauchgas von Verbrennungsprozessen oder anderen Oxidationsprozessen von Kohlenstoff mittels CO₂-Abscheidung einer Zementfabrik etc. entnommen. Die bei der Synthesereaktion freigesetzte Wärmeenergie wird zum Beispiel für eine Meerentsalzungs- oder Heizungsanlage verwendet.

DE 10 2013 010 909 B3 beschreibt die Nutzung von CO₂ aus der Kalzinierung von Kalkstein in einem Schachtofensystem unter Verwendung kohlenstoffhaltiger Energieträger und Umsetzung des CO₂ zu Methan oder Methanol mit regenerativ erzeugtem Wasserstoff aus einem separaten Elektrolyseprozess. Hauptmerkmal ist die Erhöhung der CO₂-Konzentration im überschüssigen Abgas.

US 3,817,725 A beschreibt ein Verfahren zur Herstellung eines methanhaltigen Gases, bei dem in einer endothermen Vergasungsstufe Abfälle unter Erzeugung von CO₂- und H₂-haltigem Abgas verbrannt werden und das Abgas einem Methanisierungsprozess zugeführt wird. Das im Methanisierungsprozesses erzeugte heiße methanhaltige Gas wird teilweise der Vergasungsstufe zugeführt.

Aus US 3,938,968 A ist ebenfalls ein Verfahren zur Herstellung eines methanhaltigen Gases bekannt, bei dem in einer (exothermen) Reaktion Kohlenwasserstoffe mittels angereicherten Sauerstoffs partiell oxidiert werden. Der so erzeugte COₓ- und H₂-haltige Abgasstrom wird einer (leicht exothermen) Wassergas-Shiftreaktion zu Anhebung des Wasserstoffanteils zugeführt und anschließend verschiedenen Methanisierungsstufen. Hochdruckgas, das im Prozess erhalten wird, wird unter anderem der partiellen Oxidierungsstufe zugeführt.

WO 2015/055349 A1 beschreibt die Kombination eines Kalzinierungsprozesses, eines Methanisierungsprozesses sowie eines Elektrolyseprozesses. Dabei wird aus der Elektrolyse von Wasser H₂ und O₂ erzeugt, welche dem Methanisierungsprozesses bzw. dem Kalzinierungsprozesses zugeführt werden. Eine im Methanisierungsprozess freigesetzte Wärmeenergie kann für verschiedene Prozesse im System genutzt werden, unter anderem für den Kalzinierungsprozess.

Aus EP 2 818 227 A1 ist eine ebenfalls eine Kombination eines Kalzinierungsprozesses, eines Methanisierungsprozesses sowie eines Elektrolyseprozesses bekannt, allerdings ohne Wäremerückführung des Methanisierungsprozesses. Hier wird die für die endotherme Kalzinierung benötigte Energie durch Verbrennung eines kohlenstoffhaltigen Brennstoffs mit angereichertem oder reinen Sauerstoff, der aus der Elektrolyse gewonnen werden kann, innerhalb der Brennzone des Schachtofens geliefert.

Der Erfindung liegt nun die Aufgabe zugrunde, ein Verfahren zur Erzeugung eines beispielsweise als Kraftstoff verwendbaren Kohlenwasserstoffs bereitzustellen, das einen weiter verbesserten energetischen Wirkungsgrad aufweist. Es soll ferner eine zur Ausführung des Verfahrens geeignete Anlage bereitgestellt werden.

Diese Aufgabe wird durch ein Verfahren zur Erzeugung eines Kohlenwasserstoffs sowie eine entsprechende Anlage mit den Merkmalen der unabhängigen Ansprüche gelöst. Weitere bevorzugte Ausgestaltungen der Erfindung ergeben sich aus den übrigen, in den Unteransprüchen genannten Merkmalen.

Das erfindungsgemäße Verfahren umfasst:
- einen ersten Produktionsprozess, bei dem ein Rohstoff in einer endothermen Reaktion zu einem Produkt und Kohlendioxid umgesetzt wird, und
- einen zweiten Produktionsprozess, bei dem das im ersten Produktionsprozess erzeugte Kohlendioxid in einer exothermen Reaktion mit Wasserstoff, der vorzugsweise aus erneuerbarer Energie erzeugt wird, unter Erhalt des Kohlenwasserstoffs umgesetzt wird, wobei der erste und der zweite Produktionsprozess so miteinander gekoppelt sind, dass eine im zweiten Produktionsprozess freigesetzte Wärmeenergie zumindest teilweise dem ersten Produktionsprozess zur zumindest teilweisen Deckung seines Energiebedarfs zugeführt wird, und
- einen Oxyfuel-Prozess, bei dem ein Brennstoff mittels angereicherten oder reinen Sauerstoffs verbrannt wird, wobei eine im Oxyfuel-Prozess freigesetzte Wärmeenergie zumindest teilweise dem ersten Produktionsprozess zur zumindest teilweisen Deckung seines Energiebedarfs zugeführt wird und wobei im Oxyfuel-Prozess erzeugtes Kohlendioxid dem zweiten Produktionsprozess unter Umgehung des ersten Produktionsprozesses zugeführt wird.

Ein weiterer Aspekt der vorliegenden Erfindung betrifft eine Anlage zur Erzeugung eines Kohlenwasserstoffs, umfassend:
- einen ersten Prozessreaktor, der eingerichtet ist, einen Rohstoff in einer endothermen Reaktion zu einem Produkt und Kohlendioxid umzusetzen, und
- einen zweiten Prozessreaktor, der eingerichtet ist, das im ersten Prozessreaktor erzeugte Kohlendioxid in eine exotherme Reaktion mit Wasserstoff unter Erhalt des Kohlenwasserstoffs umzusetzen, wobei der erste und der zweite Produktionsreaktor so miteinander gekoppelt sind, dass eine im zweiten Produktionsreaktor freigesetzte Wärmeenergie dem ersten Produktionsreaktor zur zumindest teilweisen Deckung seines Energiebedarfs zugeführt wird, und
- einen Oxyfuel-Reaktor, der eingerichtet ist, einen Brennstoff mittels angereicherten oder reinen Sauerstoffs zu verbrennen, eine im Oxyfuel-Prozess freigesetzte Wärmeenergie zumindest teilweise dem ersten Produktionsprozess zur zumindest teilweisen Deckung seines Energiebedarfs zuzuführen und im Oxyfuel-Reaktor erzeugtes Kohlendioxid dem zweiten Produktionsprozess unter Umgehung des ersten Produktionsprozesses zuzuführen.

Die beiden Produktionsprozesse beziehungsweise Prozessreaktoren sind erfindungsgemäß somit einerseits stofflich miteinander gekoppelt, indem einerseits das im ersten Produktionsprozess als Nebenprodukt anfallende CO₂ als Rohstoff (Edukt) dem zweiten Produktionsprozess zugeführt wird. Darüber hinaus sind die beiden Produktionsprozesse beziehungsweise Produktionsreaktoren auch thermisch miteinander gekoppelt, indem die Abwärme des exothermen zweiten Produktionsprozesses beziehungsweise -reaktors zur Deckung des Energiebedarfs des endothermen ersten Reduktionsprozesses beziehungsweise -reaktors genutzt wird. Durch diese prozessinterne thermische Kopplung der beiden Prozesse und Reaktoren wird der von außen einzuspeisende Energiebedarf weiter gesenkt und somit der energetische Wirkungsgrad des Verfahrens erhöht.

Der hier vorgestellte Ansatz eignet sich insbesondere zur großtechnischen Erzeugung eines als regenerativ zu bezeichnenden Kraftstoffs unter Verwendung von Wasserstoff aus der Elektrolyse regenerativ erzeugten Stroms. Der gemäß der Erfindung erzeugte, besonders für den Mobilitätseinsatz geeignete Energieträger, beispielsweise in Form von Methan, kann somit aufgrund seiner großtechnischen Herstellung wettbewerbsfähig gegenüber anderen regenerativen Kraftstoffen dargestellt werden. Das Verfahren kann auch als Power-to-Gas (PtG) bezeichnet werden. Anstelle von Methan kann ein Kohlenwasserstoff in flüssiger Form erzeugt werden - dieser Prozess wird auch als Power-to-Liquid (PtL) bezeichnet.

Verschiedene Ausgestaltungen des ersten, Kohlendioxid als Nebenprodukt erzeugenden Produktionsprozesses sind im Rahmen der Erfindung möglich. Gemäß einer ersten Ausgestaltung umfasst der erste Produktionsprozess eine Kalzinierung von Kalkstein, bei dem Kalkstein (hauptsächlich CaCO₃) als Rohstoff in einer endothermen Reaktion zu Kalk (CaO) als Produkt und CO₂ umgesetzt wird (siehe Gleichung 1). Kalzinierung, das auch als Kalkbrennen bezeichnet wird, erfolgt in Zementwerken, bei der Produktion von Kalk zur Herstellung von Zement und anderen Materialien. Durch Nutzung des als Nebenprodukt der Kalzinierung von Kalk anfallenden CO₂ für den zweiten Produktionsprozess werden somit sämtliche Produkte der Kalzinierung als marktfähige Produkte oder Zwischenprodukte genutzt.

CaCO₃ → CaO + CO₂ (1)

In alternativer Ausführung des Verfahrens umfasst der erste Produktionsprozess die Eisenverhüttung von Eisenerz, bei welcher Eisenerz, das verschiedene Eisenoxide (FeOₓ = Fe₂O₃, Fe₃O₄, FeO) enthalten kann, als Rohstoff in verschiedenen endothermen Reaktionen zu elementarem Eisen als Produkt und CO₂ umgesetzt wird (siehe Gleichungen 2 bis 4). Die Eisenerzverhüttung findet üblicherweise in Hochöfen statt, bei dem das Eisenerz mit Kohlenmonoxid, welches aus der simultanen Verbrennung von Kohle gewonnen wird, zu elementarem Eisen reduziert wird. Das Roheisen findet insbesondere in der Stahlproduktion Verwendung.

3Fe₂O₃ + CO → 2Fe₃O₄ + CO₂ (2)

Fe₃O4 + CO → 3FeO + CO₂ (3)

FeO + CO → 3Fe + CO₂ (4)

Beide Produktionsprozesse, Kalkbrennen und Eisenerzverhüttung, stellen technische Prozesse dar, die in großtechnischem Maßstab weltweit erfolgen und somit entsprechend hohe CO₂-Emissionen und immense Energieverbräuche aufweisen. Durch Nutzung dieser CO₂-Emissionen als Wertstoff zur Kohlenwasserstoffproduktion und durch die zumindest teilweise Deckung des immensen Energiebedarfs dieser Produktionsprozesse aus der exothermen Methanisierungsreaktion oder anderen Kohlenwasserstoff produzierenden Reaktionen wird ein erheblicher energetischer und ökologischer Nutzen erzielt.

Der erfindungsgemäße Ansatz bietet insbesondere für integrierte Eisenhütten-, Stahl- und Zementwerke eine ökonomische Option zur Reduzierung der CO₂-Emissionen und der Verbesserung der ökonomischen Bedingungen des volatilen Stahl- und Zementgeschäfts durch die Nutzung oder Vermarktung von sogenannten Koppelprodukten wie der Erzeugung von Industriesauerstoff in der Elektrolyse, der für die Stahlherstellung unmittelbar genutzt werden kann.

Im zweiten Produktionsprozess wird das aus dem ersten Produktionsprozess gewonnene CO₂ in einer exothermen Reaktion mit Wasserstoff zu einem Kohlenwasserstoff umgesetzt. Bei dem im zweiten Produktionsprozess erzeugten Kohlenwasserstoff handelt es sich insbesondere um 7 Methan CH₄, da dieses in einfacher Weise aus CO₂ durch Umsetzen mit Wasserstoff erhalten werden kann (Gleichung 5).

CO₂ + 4H₂ → CH₄ + 2H₂O (5)

Vorzugsweise erfolgt der zweite Produktionsprozess ohne externe Zufuhr eines kohlenstoffhaltigen Energieträgers, das heißt, der gesamte Kohlenstoffanteil des erzeugten Kohlenwasserstoffs stammt aus dem CO₂ des ersten Produktionsprozesses. Weiterhin ist bevorzugt, den zweiten Produktionsprozess ohne Zufuhr von Sauerstoff durchzuführen. Vorzugsweise wird für den zweiten Produktionsprozess ein Katalysator eingesetzt, wobei ein für die Fischer-Tropsch-Synthese gebräuchlicher Katalysator eingesetzt werden kann. Hier kommen insbesondere Übergangsmetalle zum Einsatz, speziell Kobalt, Eisen, Nickel und/oder Ruthenium.

In bevorzugter Ausführung des Verfahrens wird der im zweiten Produktionsprozess eingesetzte Wasserstoff aus einem Elektrolyseprozess gewonnen, insbesondere aus der Elektrolyse von Wasser (Gleichung 6). Ein solcher Elektrolyseprozess kann mit etablierten Methoden durchgeführt werden und lässt sich ohne umweltrelevante Nebenprodukte durchführen. Zudem stellt auch der bei der Elektrolyse von Wasser erzeugte Sauerstoff ein werthaltiges Produkt dar, welches extern, vorzugsweise aber intern im Verfahren genutzt werden kann.

H₂O → H₂ + ½O₂ (6)

Ein weiterer positiver Effekt der Elektrolyse ist die Stabilisierung des Stromnetzes durch die extrem hohe Aufnahmekapazität überschüssigen volatilen Stroms mittels der Elektrolyse-Anlage und die Langzeitspeicherfähigkeit in Form der hiermit erzeugten Wasserstoff- und Sauerstoff-Gase. Diese Stabilisierung lässt einen praktisch unbegrenzten Ausbau der regenerativen Stromerzeugung zu.

Eine weitere Ausführung des Verfahrens sieht vor, dass dem ersten Produktionsprozess Sauerstoff zugeführt wird, welcher dem ersten Produktionsprozesses entweder als unmittelbarer oder mittelbarer Reaktant oder als Oxidationsmittel für einen zugeführten Brennstoff, mit welchem der verbleibende Energiebedarf der endothermen Reaktion des ersten Produktionsprozesses gedeckt wird. Vorzugsweise wird auch dieser dem ersten Produktionsprozess zugeführte Sauerstoff aus einem Elektrolyseprozess gewonnen, insbesondere aus demselben bereits für die Produktion des im zweiten Produktionsprozess eingesetzten Wasserstoffs verwendeten Elektrolyseprozess (siehe oben). Somit können beide Produkte des Elektrolyseprozesses, nämlich Wasserstoff und Sauerstoff, innerhalb des erfindungsgemäßen Verfahrens genutzt werden.

Die Erfindung sieht vor, dass das Verfahren ferner einen Oxyfuel-Prozess umfasst, bei dem ein Brennstoff mittels angereicherten oder reinen Sauerstoffs verbrannt wird. Dabei kann eine im Oxyfuel-Prozess freigesetzte Wärmeenergie zumindest teilweise dem ersten Produktionsprozess zur zumindest teilweisen Deckung seines Energiebedarfs zugeführt werden und das bei dem Oxyfuel-Prozess erzeugte Kohlendioxid dem zweiten Produktionsprozess als Edukt zugeführt werden. Vorzugsweise werden sowohl die Wärme als auch das CO₂ entsprechend im Verfahren genutzt. Der Oxyfuel-Prozess zeichnet sich gegenüber herkömmlichen Verbrennungsprozessen von Brennstoffen dadurch aus, dass als Oxidationsmittel für die Verbrennung nicht Luft, sondern reiner oder zumindest angereicherter Sauerstoff verwendet wird. Aufgrund der Abwesenheit beziehungsweise geringen Konzentration von Stickstoff, Argon und anderen Bestandteilen der Luft entstehen beim Oxyfuel-Verfahren praktisch ausschließlich die Produkte CO₂ und Wasser. Somit entstehen beim Oxyfuel-Verfahren nicht nur die genannten Produkte in vergleichsweise hoher Reinheit, sondern auch aufgrund der besonders hohen Flammtemperaturen große freigesetzte Wärmemengen auf hohem Temperaturniveau. Sowohl die chemischen Produkte als auch die thermischen Produkte des Prozesses lassen sich somit sehr gut in das bestehende Verfahren integrieren und nutzen. Der Oxyfuel-Prozess wird vorzugsweise so dimensioniert, dass die in diesem Prozess erzeugte Wärmeenergie zusammen mit der im zweiten Produktionsprozess erzeugten Wärmeenergie ausreicht, um den Wärmeenergiebedarf des ersten Produktionsprozesses zu decken.

In bevorzugter Ausführung des Verfahrens wird der in dem Oxyfuel-Prozess eingesetzte Sauerstoff aus einem Elektrolyseprozess gewonnen, insbesondere aus der oben beschriebenen Wasserelektrolyse. Nach Abtrennung des Wasserstoffs aus dem Sauerstoff/Wasserstoff-Gemisch des Elektrolyseverfahrens steht der Sauerstoff mit hoher Reinheit zur Verfügung und lässt sich somit sehr gut für den Oxyfuel-Prozess einsetzen.

In bevorzugter Ausführung der Erfindung wird der für den im erfindungsgemäßen Verfahren eingesetzte Elektrolyseprozess erforderliche Strom aus regenerativen (erneuerbaren) Energien gewonnen. Hier kommt beispielsweise aus Windkraftanlagen und/oder Photovoltaikanlagen erzeugter Strom zum Einsatz. Durch die gute Speicherbarkeit der Produkte H₂ und O₂ kann der Elektrolyseprozess zudem der Stabilisierung des Stromnetzes dienen.

In einer weiteren Ausgestaltung wird Wasser, das im Abgas des zweiten Produktionsprozesses enthalten ist, zumindest teilweise dem Elektrolyseprozess zugeführt. Somit kann der externe Wasserbedarf des Verfahrens gesenkt werden.

Weiterhin kann vorgesehen sein, das Abgas des zweiten Produktionsprozesses, das üblicherweise Wasserdampf enthält, einer Dampfturbine zuzuführen, um somit nutzbare mechanische Energie unter Abtrennung des Wasserdampfes zu erzeugen. Das hierbei abgetrennte kondensierte Wasser kann mit Vorteil dem Elektrolyseprozess zugeführt werden.

Vorzugsweise umfasst das Verfahren ferner einen CO₂-Abtrennungsprozess, bei dem das aus dem ersten Produktionsprozess (und gegebenenfalls aus dem Oxyfuel-Prozess) erhaltene Kohlendioxid vor seiner Zuführung zu dem zweiten Produktionsprozess von weiteren Bestandteilen abgetrennt wird. Der Abtrennungsprozess richtet sich insbesondere nach Art des ersten Produktionsprozesses und den damit verbundenen Nebenprodukten. Grundsätzlich kann jedoch eventuell im Gasgemisch enthaltenes Kohlenmonoxid in dem dem zweiten Produktionsprozess zugeführten Eduktgas enthalten bleiben, da dieses ohne Weiteres neben dem CO₂ im zweiten Produktionsprozess in den Kohlenwasserstoff überführt werden kann. Geeignete Verfahren zum Abtrennen von CO₂ umfassen beispielsweise Gaswäscheverfahren mittels Absorption (zum Beispiel Aminwäsche), Temperaturwechseladsorption, Druckwechseladsorption, kryogene Abtrennverfahren, Membranabtrennverfahren und andere.

Im Rahmen der vorliegenden Erfindung ist es grundsätzlich möglich, den ersten und zweiten Produktionsprozess in einer gekoppelten Reaktion in einem einzigen Reaktor durchzuführen. Dabei werden dem Reaktor der Rohstoff, beispielsweise Kalkstein oder Eisenerz, Wasserstoff und Sauerstoff und gegebenenfalls weitere Edukte zugeführt, sodass diese unter Erzeugung von CO₂ als Zwischenprodukt zu dem Produkt, insbesondere Calciumoxid oder Roheisen, und dem Kraftstoff reagieren.

Vorzugsweise werden der erste und der zweite Produktionsprozess jedoch in voneinander getrennten Reaktoren durchgeführt. Der Vorteil ist hier, dass die unterschiedlichen Druck- und Temperaturniveaus, die für die jeweiligen Prozesse zu bevorzugen sind, unabhängig voneinander eingestellt werden können. Dies schließt jedoch nicht aus, dass die beiden Reaktoren in einem gemeinsamen Gehäuse untergebracht sein können.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine Anlage zur Erzeugung eines Kohlenwasserstoffs gemäß einer nicht der Erfindung zugehörigen Ausgestaltung;
- Figur 2: eine Anlage zur Erzeugung eines Kohlenwasserstoffs gemäß einer Ausgestaltung der Erfindung;
- Figur 3: eine erfindungsgemäße Anlage zur Erzeugung eines Kohlenwasserstoffs am Beispiel eines mit einem Methanisierungsprozess gekoppelten Kalzinierungsprozesses, und
- Figur 4: eine erfindungsgemäße Anlage zur Erzeugung eines Kohlenwasserstoffs am Beispiel eines mit einem Methanisierungsprozess gekoppelten Eisenhüttenprozesses.

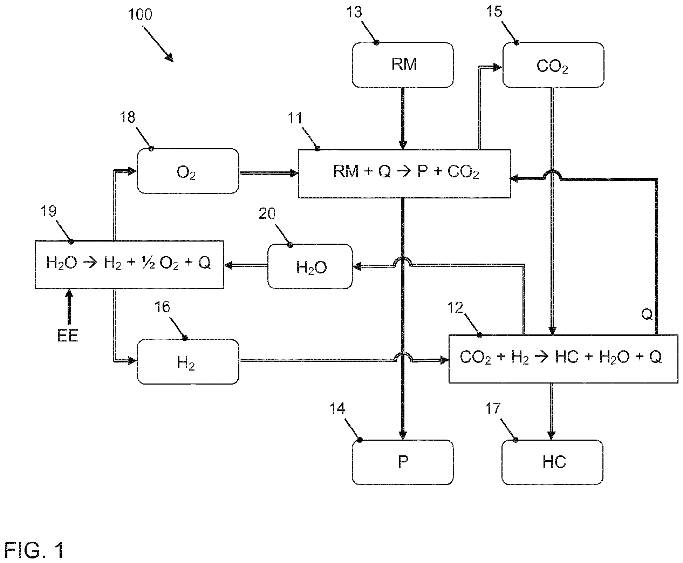

Figur 1 zeigt als Blockdiagramm eine Anlage 100 zur Erzeugung eines Kohlenwasserstoffs HC gemäß einer nicht der vorliegenden Erfindung zugehörigen Ausgestaltung.

Kern der Anlage 100 sind zwei Produktionsprozesse, nämlich ein erster Produktionsprozess 11, der in einem ersten Prozessreaktor 11 stattfindet, sowie ein zweiter Produktionsprozess 12, der in einem zweiten Prozessreaktor 12 stattfindet. Stoffströme sind in Figur 1 und den nachfolgenden Figuren als doppellinierte Pfeile dargestellt und Energieströme als einfachlinierte Pfeile.

Der erste Produktionsprozess 11 umfasst das Umsetzen eines Rohstoffs RM in einer endothermen Reaktion, also unter Verbrauch von Wärme Q, zu einem Produkt P und Kohlendioxid CO₂. Der Rohstoff RM wird aus einem Rohstoffspeicher 13 zugeführt und das Produkt P wird in einem Produktspeicher 14 (zwischen)gespeichert. Das im ersten Produktionsprozess 11 entstehende Kohlendioxid wird dem zweiten Produktionsprozess 12 zugeführt, wobei es optional in einem CO₂-Speicher 15 zwischengespeichert werden kann. In dem zweiten Produktionsprozess 12 wird das CO₂ mit Wasserstoff H₂ unter Erhalt eines Kohlenwasserstoffs HC und Wasser H₂O umgesetzt. Dieser Prozess erfolgt exotherm, also unter Freisetzung von Wärme Q. Der für die Reaktion des zweiten Produktionsprozesses 12 erforderliche Wasserstoff wird aus einem Wasserstoffspeicher 16 zugeführt. Der Kohlenwasserstoff HC als Hauptprodukt des zweiten Produktionsprozesses 12 wird in einem Kohlenwasserstoffspeicher 17 (zwischen)gespeichert.

Die im zweiten Produktionsprozess 12 freigesetzte Wärme Q wird dem ersten Produktionsprozess 11 zugeführt, um dessen Wärmebedarf zumindest teilweise zu decken. Die Übertragung der Wärmeenergie Q kann beispielsweise durch direkte Zuführung des heißen Abgases aus dem zweiten Reaktor 12 in den ersten Prozessreaktor 11 erfolgen. Alternativ kann die Abwärme über einen Wärmetauscher eines Kühlkreislaufs des zweiten Prozessreaktors 12 abgeführt und über einen weiteren Wärmetauscher des ersten Prozessreaktors 11 dort abgegeben werden. Ebenfalls ist möglich, die beiden Prozessreaktoren 11 und 12 direkt thermisch miteinander zu koppeln, beispielsweise durch unmittelbare Nachbarschaft beziehungsweise eine gemeinsame, die beiden Prozesse 11 und 12 trennende Gehäusewand. Weiterhin ist eine Zwischenspeicherung der Wärme, beispielsweise in einem sogenannten Phasenwechselmaterial (Phase-Change-Material) möglich. Dieser Ansatz ist dann von Vorteil, wenn eine absatzweise, nicht kontinuierliche Prozessführung für den Wärme liefernden Prozess 12 erfolgt.

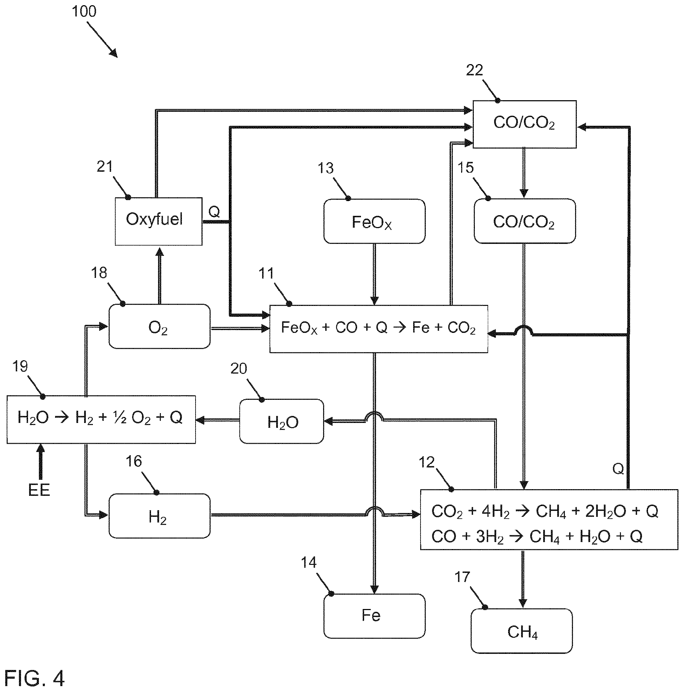

Abhängig von der Natur des ersten und zweiten Produktionsprozesses 11, 12 wird die im zweiten Prozess 12 freigesetzte Wärmeenergie Q nicht in allen Fällen ausreichen, um den Energiebedarf des ersten Produktionsprozesses 11 zu decken. Zu diesem Zweck kann vorgesehen sein, dem ersten Produktionsprozess 11 Sauerstoff O₂, der in einem Sauerstoffspeicher 18 bevorratet wird, oder Luft zuzuführen. Der Sauerstoff dient dann als Oxidationsmittel für einen Brennstoff, beispielsweise Kohle oder dergleichen, um die erforderliche Reaktionswärme durch diesen zusätzlichen Verbrennungsprozess zu decken. Daneben kann der Sauerstoff jedoch als mittelbarer oder unmittelbarer Reaktant für den ersten Produktionsprozess 11 dienen, wie etwa bei der Verhüttung von Eisenerz, wo der Sauerstoff zur Verbrennung von Kohle zur intermediären Erzeugung von Kohlenmonoxid zur Reaktion von Eisenoxid dient (siehe Figur 4).

Die in Figur 1 gezeigte Anlage 100 umfasst ferner einen Elektrolyseprozess 19, insbesondere einen Wasserelektrolyseprozess. Hierbei wird Wasser H₂O unter Zuführung von elektrischer Energie EE in seine Bestandteile Wasserstoff H₂ und Sauerstoff O₂ zerlegt. Die Zwischenspeicherung dieser Gase erfolgt in den bereits erwähnten Speichern 16 und 18. Die Wasserelektrolyse erfolgt nach bekannten Verfahren im intermittierenden Betrieb und mit modularem Aufbau der Elektrolyseanlage. Als Strom EE wird vorzugsweise volatiler, regenerativ erzeugter Strom verwendet, beispielsweise aus Windkraftanlagen und/oder Photovoltaikanlagen. Das für die Elektrolyse 19 eingesetzte Wasser kann gemäß der in Figur 1 dargestellten Anlage zumindest teilweise aus dem zweiten Produktionsprozess 12 gewonnen werden. Beispielsweise kann das Wasser aus dem Abgas des Reaktors 12 durch einen Wasserabscheider oder mittels einer nachgeschalteten Dampfturbine mit integriertem Wasserabscheider gewonnen werden. Alternativ oder zusätzlich kann das Wasser aus dem öffentlichen Wasserversorgungsnetz bezogen werden.

Abhängig von der Art des ersten Produktionsprozesses 11 kann ferner eine CO₂-Trennstufe vorgesehen sein (siehe nachfolgende Figuren).

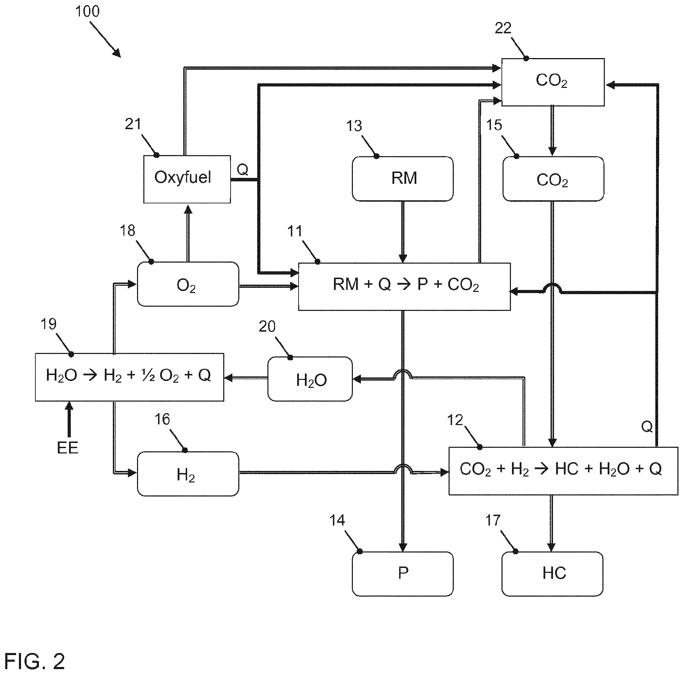

Figur 2 zeigt eine Anlage zur Erzeugung eines Kohlenwasserstoffs gemäß einer Ausgestaltung der Erfindung, wobei die gleichen Elemente wie in Figur 1 mit den gleichen Bezugszeichen bezeichnet sind. Hierzu gelten die Ausführungen zu Figur 1 entsprechend.

Die Anlage gemäß Figur 2 unterscheidet sich von der aus Figur 1 durch Einbindung eines Oxyfuel-Prozesses, welcher in einem Oxyfuel-Reaktor 21 beziehungsweise einem Oxyfuel-Kraftwerk 21 stattfindet. Bei dem Oxyfuel-Prozess 21 erfolgt die Verbrennung eines Brennstoffs mittels reinen oder (gegenüber dem Sauerstoffgehalt von Luft) angereicherten Sauerstoffs. Im vorliegenden Beispiel wird der aus der Wasserelektrolyse 19 stammende Sauerstoff O₂ aus dem Sauerstoffspeicher 18 dem Oxyfuel-Reaktor 21 zugeführt und in diesem umgesetzt. Als Brennstoff des Oxyfuel-Prozesses 21 kann grundsätzlich ein beliebiger fester, flüssiger oder gasförmiger Brennstoff eingesetzt werden. Gemäß einer Ausführung der Erfindung wird der aus dem zweiten Produktionsprozess 12 gewonnene Kohlenwasserstoff 17 teilweise für den Oxyfuel-Prozess 21 eingesetzt.

Das chemische Hauptprodukt des Oxyfuel-Prozesses 21 ist CO₂. Abhängig von dem hier eingesetzten Brennstoff können andere Bestandteile in dem Abgas des Oxyfuel-Kraftwerks 21 enthalten sein, beispielsweise Kohlenmonoxid CO, Stickoxide NOₓ, Schwefeloxide SOₓ und andere. Auch das Abgas des ersten Produktionsprozesses 11 kann neben CO₂ weitere Bestandteile wie CO, NOₓ oder SOₓ, enthalten. Sowohl das Abgas des ersten Produktionsprozesses 11 sowie des Oxyfuel-Prozesses 21 werden in der in Figur 2 gezeigten Anlage 100 in einer CO₂-Trennstufe 22 von dem CO abgetrennt, bevor dieses dem zweiten Produktionsprozess 12 zugeführt wird.

Die im Reaktor 11 stattfindende Kalzinierung läuft bei einer Temperatur von ca. 1200°C ab. Wie bereits geschildert, wird dieses Temperaturniveau zumindest teilweise durch die Abwärme des zweiten Produktionsprozesses 12 und gegebenenfalls der Abwärme des Oxyfuel-Prozesses 21 dargestellt. Möglich ist auch die Einbindung von Fremdenergie, beispielsweise durch einen Lichtbogenprozess oder durch einen H₂/O₂-Brenner ohne Zufuhr von kohlenstoffhaltigen Energieträgern.

Der zweite Produktionsprozess 12, der hier als Methanisierungsprozess ausgeführt ist, sieht die Umsetzung des aus der Kalzinierung 11 stammenden CO₂ mit dem aus der Elektrolyse 19 stammenden Wasserstoff H₂ zu Methan CH₄ und Wasserdampf vor. Als Reaktor 12 kann hier beispielsweise ein Wirbelschichtreaktor unter Nutzung eines Fischer-Tropsch-Katalysators eingesetzt werden. Außer dem aus dem Kalzinierungsprozess 11 und gegebenenfalls Oxyfuel-Prozess 21 erzeugten CO₂ erfolgt keine Zufuhr eines kohlenstoffhaltigen Energieträgers für die Methanisierungsreaktion. Das in dieser Reaktion erhaltene Methan CH₄ kann gegebenenfalls nach Reinigung von Schadstoffen als Brennstoff für Heizungen oder als Kraftstoff für Verbrennungsmotoren verwendet werden. Gegebenenfalls können hier Aufarbeitungen erfolgen, um eine Anpassung an eine erforderliche Qualität bezüglich des Heizwerts, Wasserstoffgehalts, Schadstoffgehalts etc. vorzunehmen.

Figur 4 zeigt eine erfindungsgemäße Anlage 100 am Beispiel eines mit einem Methanisierungsprozess 12 gekoppelten Eisenhüttenprozesses 11. In diesem Beispiel umfasst somit der erste Produktionsprozess 11 einen Hochofenprozess, in dem die Verhüttung von Eisenerz erfolgt, wobei hier FeOₓ für die verschiedenen, in Eisenerzen vorliegenden Eisenoxide steht. Bei der Eisenerzverhüttung wird in bekannter Weise das Eisenerz FeOₓ in einem Hochofen mit Kohlenmonoxid, das üblicherweise aus der Verbrennung von Kohle mit Luft beziehungsweise hier angereichertem Sauerstoff erzeugt wird, zu Roheisen Fe und Kohlendioxid CO₂ umgesetzt. Das hier erhaltene Roheisen Fe wird insbesondere zur Stahlproduktion oder dergleichen verwendet.

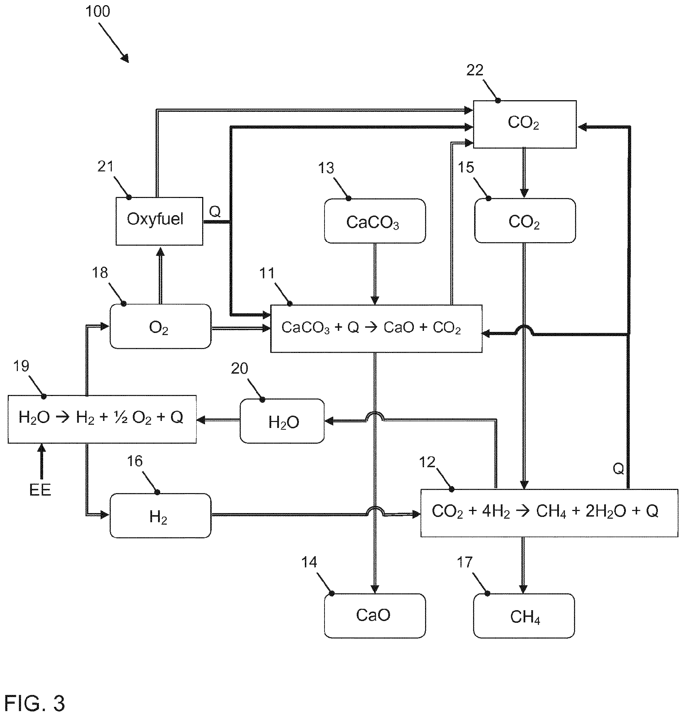

Das Abgas des Hochofenprozesses enthält neben dem CO₂ auch nicht umgesetztes Kohlenmonoxid CO. Beide Bestandteile werden in einer Trennstufe 22 von den übrigen Bestandteilen des Abgases abgetrennt und dem Methanisierungsprozess 12 als Edukte zugeführt. Die Durchführung des Methanisierungsprozesses entspricht dem in Figur 3. Beide Edukte, CO₂ und CO, werden hier durch Umsetzung mit dem aus der Elektrolyse 19 stammenden Wasserstoff zu Methan CH₄ umgesetzt.

Nachfolgend erfolgen einige quantitative Betrachtungen des erfindungsgemäßen Gesamtprozesseses am Beispiel der Kopplung Methanisierung und Kalzination:
- Basis ist ein Zementwerk mittlerer Größe mit CO₂-Emissionen von 1 Mio. Jahrestonnen (t/a). Von 1 Mio. t CO₂-Gesamtemissionen stammen konservativ angenommen 50 % aus den Prozessemissionen der Kalzination des Kalksteins CaCO₃. Diese 0,5 Mio. t/a CO₂ führen zu 0,636 Mio. t/a CaO für das Zementwerk und ermöglichen auf Basis der Kohlenstoffbilanz und der Stöchiometrie die Herstellung von 0,18 Mio. t/a Methan CH₄. Diese Methanmenge hat einen Gesamt-Heizwert von 2,54 TWh/a basierend auf dem spezifischen Heizwert von 14 MWh/t CH₄.
- Nach der Elektrolyse-Reaktionsgleichung 2H₂O → 2H₂ + O₂ entstehen 2 mol H₂ mit einer Bildungsenthalpie von 0,079 kWh/(2 mol H₂) beziehungsweise 39,5 MWh/(t H₂). Die Methanisierung benötigt nach der Reaktionsgleichung CO₂ + 4H₂ → CH₄ + 2H₂O jedoch 4 mol H₂, um 1 mol CH₄ herzustellen. Daraus ergibt sich ein theoretischer Mindestbedarf von 2 x 0,045 = 0,09 Mio. t/a H₂ mit einem Heizwert von 3,2 TWh/a. Der reale elektrische Energiebedarf für eine entsprechend leistungsfähige Elektrolyseanlage erreicht auf Basis eines Stromverbrauchs von 53,6 MWh/t H₂ beziehungsweise einem Elektrolyseur-Wirkungsgrad von 74 % 4,3 TWh/a. Bei Wirkungsgraden der Elektrolyse unter 70 % ergibt sich ein elektrischer Energiebedarf von über 4,6 TWh/a.
- Bei ausschließlich elektrischer Bereitstellung des Energiebedarfs zur Zementherstellung von 830-908 kWh/t Zement ergibt sich ein Kostenblock auf Basis eines Outputs von 0,7 Mio. t Zement und eines mittleren Lieferpreises von 50 EUR/MWh von etwa 30 Mio. EUR. Der Erlös für den Zement beläuft sich bei einem Zementpreis zwischen 127 und 139 EUR/t auf ca. 100 Mio. EUR.
- Die Kosten für den elektrischen Energiebedarf in einem weiterentwickelten Elektrolyseur 53,6 MWh/t H₂ ergeben für 0,09 Mio. t H₂ und dem resultierenden Energiebedarf von 4,3 TWh/a den dominierenden Kostenblock von 215 Mio. EUR.
- Die Kosten des Prozesswassers für die Elektrolyse (0,8 Mio. t) werden auf Basis von 2 EUR/t H₂O mit 1,6 Mio. EUR geschätzt (ohne Wasserrückgewinnung aus dem Prozess).
- Der als Industriegas vermarktbare Sauerstoff fällt in Höhe von 0,7 Mio. t an und stellt bei Sauerstoffpreisen von 100 EUR/t O₂ ein Erlöspotenzial von 70 Mio. EUR, bei 140 EUR/t von ca. 100 Mio. EUR dar. Hauptverbraucher für Industrie-Sauerstoff ist die Stahlindustrie. Die derzeit häufigste Produktionsmethode für die wichtigsten Industriegase aus der Luft ist die kryotechnische Luftzerlegung, die auch wertvolle Edel- und Spurengase liefert. Insofern kann der Elektrolyse-Sauerstoff die Stahlindustrie im Hinblick auf ihre CO₂-Verpflichtungen bei Nutzung des Elektrolyse-EE-Sauerstoffs aber auch wesentlich entlasten.
- Die Methanisierung weist ein Output von 0,18 Mio. t CH₄ auf und führt zu einem Methan-Erlöspotenzial von 65,5 Mio. EUR bei einem angenommenen Erdgaspreis von 26 EUR/MWh (0,36 EUR/kg) oder zu einem Potenzial von 151 Mio. EUR bei einem angenommenen Gaspreis von 60 EUR/MWh (0,84 EUR/kg). Das Methan kann Tankstellen für mobile Anwendungen zugeführt werden.
- Die derzeit bestehende Gesamtspeicherkapazität der deutschen Erdgasinfrastruktur von ca. 200 TWh sollte für die Nutzung als Speicher für die volatilen Überschuss-Strommengen in Form von EE-PtG-H₂ (PtG = Power-to-Gas) und EE-PtG-Methan ausreichend sein.
- Aufgrund des modulartigen Aufbaus von Elektrolyseanlagen und der systembedingten Entkoppelung von Elektrolyse und der Methanisierung steht die gegenüber Anlagen für komplexe verfahrenstechnische Prozesse relativ leicht zu erweiternde und unter anderem durch Zuschaltung von Modulen instationär arbeitsfähige Elektrolyseanlage als Überschussstromsenke zur Verfügung. Der erzeugte Wasserstoff kann sowohl in das Erdgasnetz eingespeist werden als auch für die weitere Methanisierung eingesetzt werden. Die Kapazität der Methanisierung wird vom CO₂-Angebot der Zementherstellung bestimmt.
- Zu dem hier ermittelten Bedarf von 4,3 TWh für die Elektrolyse und beispielsweise einer Übertragung mittels einer Doppelsystem-380 kV-Drehstromleitung mit 3,5 GW Übertragungskapazität ergibt sich eine minimale Einspeisezeit von ca. 1200 h bei entsprechender Aufnahmefähigkeit der Elektrolyse-Anlage von mindestens 3,5 GW. Bei Einhaltung der Überbrückungszeit während der Überschussphasen des EE-Stroms von 14 Tagen oder 336 h ergeben sich etwa vierfache Leitungskapazität und vierfache Aufnahmefähigkeit des Elektrolyseurs mit jeweils notwendigen 15 GW. Bei einem geschätzten Gesamtspeicherbedarf von 15 TWh und 14 Tagen Überbrückungszeit würden 3 bis 4 derartige Anlagen in Deutschland mit einer Gesamtkapazität von 45 GW benötigt. Eine ausreichend dimensionierte Elektrolyseanlage kann in der verbrauchernahen Koppelung mit dem Erdgasnetz anfallende Überschussmengen kurzfristig aufnehmen.
- Die (zwangsläufig mit Unsicherheiten behaftete) Gesamtbilanz lässt die Wirtschaftlichkeit des Verfahrens aussichtsreich erscheinen.
- Als ein präferierter Nutzungspfad wird das erzeugte EE-PtG-Methan (PtG = Power-to-Gas) gegebenenfalls unter Anreicherung mit Wasserstoff (derzeit für Verbrennungsmotoren auf 2 Vol. % begrenzt) unter Einhaltung der Erdgasnetzspezifikationen dazu genutzt, um bei einem angenommenen spezifischen Gasverbrauch/Fahrzeug von 3 kg Methan/Wasserstoff-Gemisch/100 km mit einem Heizwert von mehr als 15 kWh/kg Mischgas ca. 400.000 Gasfahrzeuge bei einer Jahresfahrleistung von 15.000 km zu versorgen. Damit kann zum Beispiel der PKW-Bestand der Stadt und der Region Hannover nachhaltig betrieben werden.

Vorteile der Erfindung:
- Das vorliegende Konzept der Erzeugung von EE-PtG-Methan durch EE-Strom und unter Nutzung der im Kalkstein gespeicherten CO₂-Emissionen, die bei der Zementherstellung freigesetzt und für EE-Methan sinnvoll genutzt werden, kombinieren das Beste aus den drei Welten Strom, Gas und Mobilität und auch der Wasserstoff findet seinen Platz in diesem System, ohne eine zum Erdgasnetz zusätzliche und aufwendige Wasserstoffinfrastruktur entwickeln und aufbauen zu müssen. Die mit Nachteilen verbundene Einlagerung von CO₂ (Carbon Capture and Storage, CCS) wird hiermit obsolet
- Die nachhaltige Mobilität, die bisher auf Nischenansätze beschränkt war, findet nun eine massen- und systemkompatible erneuerbare Energiebasis, deren Nutzung im Fahrzeug erprobt ist.
- Der Ausbau einschließlich des Repowering der regenerativen Energien Wind und Photovoltaik kann im Hinblick auf die sinnvolle Nutzung der eingesammelten Energie unter Einsatz der hier gezeigten Speichermöglichkeiten weiter vorangetrieben werden.
- Wirtschaftliche Faktoren für das Konzept sind nicht nur die Kurzzeit-Speicherung von Strom-Überschussmengen im Erdgasnetz in Form von EE-H₂ oder EE-Methan und die Vermarktung dieser der Kraftstoffe (insbesondere EE-PtG-Methan mit erhöhtem H₂-Gehalt). Durch das Potenzial am Kraftstoffmarkt steht eine Nutzungsoption für den Kraftstoffanbieter oder den Fahrzeughersteller zur Verfügung, um die EE-PtG-Methan-Mengen und Anteile der EE-PtG-H₂-Mengen im Rahmen CO₂-Minderungsstrategie als Dekarbonisierungsmaßnahme zu nutzen.
- Die verfügbaren CO₂-Mengen sind aufgrund der insbesondere in Deutschland vorhandenen Kapazitäten der Grundstoffindustrien, insbesondere der Zementherstellung und der Stahlindustrie, vorhanden.
- So könnten alle Zementwerke in Deutschland auf Basis von 10 Mio. t CO₂ als Prozessemissionen den nachhaltigen Betrieb von 8 Mio. Gasfahrzeugen und die integrierten Eisenhütten- und Stahlwerke auf Basis von angenommenen 25 Mio. t CO₂ als unvermeidbare Prozessemissionen den nachhaltigen Betrieb von 20 Mio. Gasfahrzeugen auf Jahresbasis ermöglichen.

### Bezugszeichenliste

- 100: Anlage zur Erzeugung eines Kohlenwasserstoffs
- 11: erster Produktionsprozess / erster Prozessreaktor / Kalzinierung / Eisenverhüttung
- 12: zweiter Produktionsprozess / zweiter Prozessreaktor / Methanisierung
- 13: Rohstoffspeicher
- 14: Produktspeicher
- 15: CO₂-Speicher
- 16: Wasserstoffspeicher
- 17: Kohlenwasserstoffspeicher / Methanspeicher
- 18: Sauerstoffspeicher
- 19: Elektrolyseprozess
- 20: Wasserspeicher
- 21: Oxyfuel-Prozess / Oxyfuel-Reaktor
- 22: CO₂-Trennstufe oder CO/CO₂-Trennstufe

- EE: elektrischer Strom aus regenerierbarer/erneuerbarer Energie
- Q: Wärme
- RM: Rohstoff / Rohmaterial
- P: Produkt
- CH₄: Methan
- CO₂: Kohlendioxid
- HC: Kohlenwasserstoff
- H₂: Wasserstoff
- O₂: Sauerstoff
- H₂O: Wasser CaCO₃Calciumcarbonat / Kalkstein
- CaO: Calciumoxid / gebrannter Kalk
- Fe: Eisen (Roheisen)
- FeOₓ: Eisenoxide (Eisenerz)

## Patentansprüche

1. Verfahren zur Erzeugung eines Kohlenwasserstoffs (HC), umfassend:
- einen ersten Produktionsprozess (11), bei dem ein Rohstoff (RM) in einer endothermen Reaktion zu einem Produkt (P) und Kohlendioxid (CO₂) umgesetzt wird, und
- einen zweiten Produktionsprozess (12), bei dem das im ersten Produktionsprozess (11) erzeugte Kohlendioxid (CO₂) in einer exothermen Reaktion mit Wasserstoff (H₂) unter Erhalt des Kohlenwasserstoffs (HC) umgesetzt wird, wobei der erste und der zweite Produktionsprozess (11, 12) so miteinander gekoppelt sind, dass eine im zweiten Produktionsprozess (12) freigesetzte Wärmeenergie (Q) dem ersten Produktionsprozess (11) zur zumindest teilweisen Deckung seines Energiebedarfs zugeführt wird,
**gekennzeichnet durch**
- einen Oxyfuel-Prozess (21), bei dem ein Brennstoff mittels angereicherten oder reinen Sauerstoffs (O₂) verbrannt wird, wobei eine im Oxyfuel-Prozess (21) freigesetzte Wärmeenergie (Q) zumindest teilweise dem ersten Produktionsprozess (11) zur zumindest teilweisen Deckung seines Energiebedarfs zugeführt wird und wobei im Oxyfuel-Prozess (21) erzeugtes Kohlendioxid (CO₂) dem zweiten Produktionsprozess (12) unter Umgehung des ersten Produktionsprozesses (11) zugeführt wird.

2. Verfahren nach Anspruch 1, **dadurch gekennzeichnet, dass** der im zweiten Produktionsprozess (12) eingesetzte Wasserstoff (H₂) aus einem Elektrolyseprozess (19) gewonnen wird.

3. Verfahren nach Anspruch 1 oder 2, **dadurch gekennzeichnet, dass** dem ersten Produktionsprozess (11) Sauerstoff (O₂) als Reaktant oder als Oxidationsmittel für einen Brennstoff zugeführt wird und der Sauerstoff (O₂) aus einem Elektrolyseprozess (19) gewonnen wird.

4. Verfahren nach einem der Ansprüche 1 bis 3, **dadurch gekennzeichnet, dass** der in dem Oxyfuel-Prozess (21) eingesetzte Sauerstoff (O₂) aus einem Elektrolyseprozess (19) gewonnen wird.

5. Verfahren nach einem der Ansprüche 2 bis 4, **dadurch gekennzeichnet, dass** der Elektrolyseprozess (19) die Elektrolyse von Wasser (H₂O), insbesondere unter Verwendung von Strom aus regenerativen Energien, umfasst.

6. Verfahren nach einem der vorhergehenden Ansprüche, ferner umfassend einen CO₂-Abtrennungsprozess (22), bei dem das Kohlendioxid (CO₂) vor Zuführung zu dem zweiten Produktionsprozess (12) von weiteren Bestandteilen abgetrennt wird.

7. Verfahren nach einem der Ansprüche 2 bis 5, **dadurch gekennzeichnet, dass** in einem Abgas des zweiten Produktionsprozesses (12) enthaltenes Wasser zumindest teilweise dem Elektrolyseprozess (19) zugeführt wird.

8. Verfahren nach einem der vorhergehenden Ansprüche, **dadurch gekennzeichnet, dass** der erste Produktionsprozess (11)
- eine Kalzinierung von Kalkstein umfasst, bei dem Kalkstein (CaCO₃) als Rohstoff (RM) in einer endothermen Reaktion zu Kalk (CaO) als Produkt (P) und Kohlendioxid (CO₂) umgesetzt wird, oder
- eine Verhüttung von Eisenerz umfasst, bei der Eisenerz als Rohstoff (RM) in einer endothermen Reaktion zu Roheisen als Produkt (P) und Kohlendioxid (CO₂) umgesetzt wird.

9. Anlage (100) zur Erzeugung eines Kohlenwasserstoffs (HC), umfassend:
- einen ersten Prozessreaktor (11), der eingerichtet ist, einen Rohstoff (RM) in einer endothermen Reaktion zu einem Produkt (P) und Kohlendioxid (CO₂) umzusetzen, und
- einen zweiten Prozessreaktor (12), der eingerichtet ist, das im ersten Produktionsprozess (11) erzeugte Kohlendioxid (CO₂) in einer exothermen Reaktion mit Wasserstoff (H₂) unter Erhalt des Kohlenwasserstoffs (HC) umzusetzen, wobei der erste und der zweite Prozessreaktor (11, 12) so miteinander gekoppelt sind, dass eine im zweiten Prozessreaktor (12) freigesetzte Wärmeenergie (Q) dem ersten Prozessreaktor (11) zur zumindest teilweisen Deckung seines Energiebedarfs zugeführt wird,
**gekennzeichnet durch**
- einen Oxyfuel-Reaktor (21), der eingerichtet ist, einen Brennstoff mittels angereicherten oder reinen Sauerstoffs (O₂) zu verbrennen, eine im Oxyfuel-Prozess (21) freigesetzte Wärmeenergie (Q) zumindest teilweise dem ersten Produktionsprozess (11) zur zumindest teilweisen Deckung seines Energiebedarfs zuzuführen und im Oxyfuel- Reaktor (21) erzeugtes Kohlendioxid (CO₂) dem zweiten Produktionsprozess (12) unter Umgehung des ersten Produktionsprozesses (11) zuzuführen.

## Claims

1. Method for producing a hydrocarbon (HC), comprising:
- a first production process (11), in which a raw material (RM) is converted to a product (P) and carbon dioxide (CO₂) in an endothermic reaction, and
- a second production process (12), in which the carbon dioxide (CO₂) produced in the first production process (11) is reacted with hydrogen (H₂) in an exothermic reaction to obtain the hydrocarbon (HC), wherein the first and the second production process (11, 12) are coupled to each other such that thermal energy (Q) released in the second production process (12) is supplied to the first production process (11) to at least partially cover its energy requirement,
**characterized by**
- an oxy-fuel process (21), in which a fuel is combusted using enriched or pure oxygen (O₂), wherein at least some of the thermal energy (Q) released in the oxy-fuel process (21) is supplied to the first production process (11) to at least partially cover its energy requirement and wherein carbon dioxide (CO₂) produced in the oxy-fuel process (21) is fed to the second production process (12) bypassing the first production process (11).

2. Method according to Claim 1, **characterized in that** the hydrogen (H₂) used in the second production process (12) is obtained from an electrolysis process (19).

3. Method according to Claim 1 or 2, **characterized in that** oxygen (O₂) as reactant or as oxidizing agent for a fuel is fed to the first production process (11) and the oxygen (O₂) is obtained from an electrolysis process (19).

4. Method according to any of Claims 1 to 3, **characterized in that** the oxygen (O₂) used in the oxy-fuel process (21) is obtained from an electrolysis process (19).

5. Method according to any of Claims 2 to 4, **characterized in that** the electrolysis process (19) comprises the electrolysis of water (H₂O), particularly using current from renewable energy sources.

6. Method according to any of the preceding claims, further comprising a CO₂ separation process (22), in which the carbon dioxide (CO₂) is separated from other constituents prior to feeding to the second production process (12).

7. Method according to any of Claims 2 to 5, **characterized in that** water present in an offgas of the second production process (12) is fed at least partially to the electrolysis process (19).

8. Method according to any of the preceding claims, **characterized in that** the first production process (11)
- comprises calcination of limestone, in which limestone (CaCO₃) as raw material (RM) is converted to lime (CaO) as product (P) and carbon dioxide (CO₂) in an endothermic reaction, or
- comprises smelting iron ore, in which iron ore as raw material (RM) is converted to crude iron as product (P) and carbon dioxide (CO₂) in an endothermic reaction.

9. Apparatus (100) for producing a hydrocarbon (HC), comprising:
- a first process reactor (11), which is configured to convert a raw material (RM) to a product (P) and carbon dioxide (CO₂) in an endothermic reaction, and
- a second process reactor (12), which is configured to react the carbon dioxide (CO₂) produced in the first production process (11) with hydrogen (H₂) in an exothermic reaction to obtain the hydrocarbon (HC), wherein the first and the second process reactor (11, 12) are coupled to each other such that thermal energy (Q) released in the second process reactor (12) is supplied to the first process reactor (11) to at least partially cover its energy requirement,
**characterized by**
- an oxy-fuel reactor (21), which is configured to combust a fuel using enriched or pure oxygen (O₂), to supply at least some of the thermal energy (Q) released in the oxy-fuel process (21) to the first production process (11) to at least partially cover its energy requirement and to feed carbon dioxide (CO₂) produced in the oxy-fuel reactor (21) to the second production process (12) bypassing the first production process (11).

## Revendications

1. Procédé de production d'un hydrocarbure (HC), comprenant:
- un premier processus de production (11), dans lequel on convertit une matière première (RM) par une réaction endothermique en un produit (P) et en dioxyde de carbone (CO₂), et
- un deuxième processus de production (12), dans lequel on fait réagir le dioxyde de carbone (CO₂) produit dans le premier processus de production (11) par une réaction exothermique avec de l'hydrogène (H₂) avec obtention d'un hydrocarbure (HC), dans lequel le premier et le deuxième processus de production (11, 12) sont couplés l'un à l'autre de telle manière qu'une énergie thermique (Q) dégagée dans le deuxième processus de production (12) soit fournie au premier processus de production (11) pour la couverture au moins partielle de ses besoins en énergie,
**caractérisé par**
- un processus oxy-fuel (21), dans lequel on brûle un combustible au moyen d'oxygène enrichi ou pur (O₂), dans lequel on fournit une énergie thermique (Q) dégagée dans le processus oxy-fuel (21) au moins en partie au premier processus de production (11) pour la couverture au moins partielle de ses besoins en énergie et dans lequel on fournit du dioxyde de carbone (CO₂) produit dans le processus oxy-fuel (21) au deuxième processus de production (12) en contournant le premier processus de production (11).

2. Procédé selon la revendication 1, **caractérisé en ce que** l'on récupère l'hydrogène (H₂) utilisé dans le deuxième processus de production (12) à partir d'un processus d'électrolyse (19).

3. Procédé selon la revendication 1 ou 2, **caractérisé en ce que** l'on fournit au premier processus de production (11) de l'oxygène (O₂) comme réactif ou comme agent oxydant pour un combustible et on récupère l'oxygène (O₂) à partir d'un processus d'électrolyse (19) .

4. Procédé selon l'une quelconque des revendications 1 à 3, **caractérisé en ce que** l'on récupère l'oxygène (O₂) utilisé dans le processus oxy-fuel (21) à partir d'un processus d'électrolyse (19).

5. Procédé selon l'une quelconque des revendications 2 à 4, **caractérisé en ce que** le processus d'électrolyse (19) comprend l'électrolyse de l'eau (H₂O), en particulier avec utilisation de courant provenant d'énergies de régénération.

6. Procédé selon l'une quelconque des revendications précédentes, comprenant en outre un processus de séparation de CO₂ (22), dans lequel on sépare le dioxyde de carbone (CO₂) d'autres constituants avant la fourniture au deuxième processus de production (12).

7. Procédé selon l'une quelconque des revendications 2 à 5, **caractérisé en ce que** l'on fournit au moins en partie au processus d'électrolyse (19) de l'eau contenue dans des gaz d'échappement du deuxième processus de production (12).

8. Procédé selon l'une quelconque des revendications précédentes, **caractérisé en ce que** le premier processus de production (11)
- comprend une calcination de calcaire, dans laquelle on convertit du calcaire (CaCO₃) comme matière première (RM) par une réaction endothermique en chaux (CaO) comme produit (P) et en dioxyde de carbone (CO₂) , ou
- comprend un traitement de minerai de fer, dans lequel on convertit du minerai de fer comme matière première (RM) par une réaction endothermique en fonte brute comme produit (P) et en dioxyde de carbone (CO₂).

9. Installation (100) pour la production d'un hydrocarbure (HC), comprenant:
- un premier réacteur de processus (11), qui est conçu pour convertir une matière première (RM) par une réaction endothermique en un produit (P) et en dioxyde de carbone (CO₂), et
- un deuxième réacteur de processus (12), qui est conçu pour faire réagir le dioxyde de carbone (CO₂) produit dans le premier processus de production (11) par une réaction exothermique avec de l'hydrogène (H₂) avec obtention de l'hydrocarbure (HC), dans lequel le premier et le deuxième réacteurs de processus (11, 12) sont couplés l'un à l'autre de telle manière qu'une énergie thermique (Q) dégagée dans le deuxième réacteur de processus (12) soit fournie au premier réacteur de processus (11) pour la couverture au moins partielle de ses besoins en énergie,
**caractérisée par**
- un réacteur oxy-fuel (21), qui est conçu pour brûler un combustible au moyen d'oxygène enrichi ou pur (O₂), fournir une énergie thermique (Q) dégagée dans le processus oxy-fuel (21) au moins en partie au premier processus de production (11) pour la couverture au moins partielle de ses besoins en énergie et fournir du dioxyde de carbone (CO₂) produit dans le réacteur oxy-fuel (21) au deuxième processus de production (12) en contournant le premier processus de production (11).
